# EUROPÄISCHE PATENTSCHRIFT

(11) **EP 1 403 240 B1**
(45) Veröffentlichungstag und Bekanntmachung des Hinweises auf die Patenterteilung: **21.07.2004**
(21) Anmeldenummer: 03020442.4
(22) Anmeldetag: 11.09.2003
(51) Int. Cl.: C07C 51/29, C07C 57/20, C07C 57/18, C07C 57/22

(54) **Verfahren zur Herstellung von Alkincarbonsäuren durch Oxidation von Alkinalkoholen**
Process for the preparation of acetylenic carboxylic acids by oxidation of acetylenic alcohols
Procédé pour la préparation d'acides carboxyliques acetyleniques par oxidation d'alcools acetyleniques

(30) Priorität: 25.09.2002 DE 10244633
(43) Veröffentlichungstag der Anmeldung: 31.03.2004
(73) Patentinhaber: Consortium für elektrochemische Industrie GmbH, 81379 München (DE)
(72) Erfinder: Stohrer, Jürgen Dr., 82049 Pullach (DE); Fritz-Langhals, Elke Dr., 85521 Ottobrunn (DE); Brüninghaus, Christian, 82008 Unterhaching (DE)
(74) Vertreter: Schuderer, Michael, Dr.

(56) Entgegenhaltungen:
- EP-A- 1 336 599
- WO-A-99/52849
- P.L. ANELLI ET AL: J. ORG. CHEM., Bd. 52, Nr. 12, 1987, Seiten 2559-2562, XP002258217

## Beschreibung

Die vorliegende Erfindung beschreibt ein Verfahren zur Oxidation von Alkinalkoholen (Alkinolen) zu Alkincarbonsäuren (Alkinsäuren).

Alkinsäuren sind wichtige Synthesebausteine. Von besonderer Bedeutung sind Propiolsäure und Acetylendicarbonsäure, die zum Aufbau von Ringen in Cycloadditionen, insbesondere Diels-Alder-Reaktionen und 1,3-dipolaren Cycloadditionen, und bei nucleophilen Additionsreaktionen eingesetzt werden (Übersicht in Ullmann' Encyclopedia, 6^{th} Edition, 2001 electronic release; "Carboxylic acids, aliphatic 5.2").

Die Oxidation von Alkinolen zu Alkinsäuren ist im Stand der Technik beschrieben worden (Übersichten in Ullmann' Encyclopedia, 6^{th} Edition, 2001 electronic release; "Carboxylic acids, aliphatic 5.2"; Houben-Weyl Band V/2a, 4. Auflage 1977, "Alkine").

So wird beispielsweise Propiolsäure durch anodische Oxidation von Propargylalkohol erhalten (Wolf, Chem. Ber. 1954, 87, 668). Ebenso erhält man Acetylendicarbonsäure durch anodische Oxidation von 2-Butin-1,4-diol. Das elektrochemische Verfahren hat aber den Nachteil der Verwendung von Bleidioxid-Anoden, die zur Kontamination der Elektrolyten mit Bleiionen führen, und ist generell nur mit großen Investitionen in der Produktion umsetzbar. Die als Nebenreaktion ablaufende Decarboxylierung des Produkts führt zudem zur technisch unerwünschten Entstehung großer Mengen an CO₂ und Acetylen, die zu entsorgen sind. Ferner sind die Ausbeuten im Fall der Propiolsäure relativ gering (unter 50%).

Die analoge anodische Oxidation an Nickeloxid-Anoden (Kaulen und Schäfer, Tetrahedron 1982, 38, 3299) erfordert geringe Stromdichten und sehr große Elektrodenoberflächen, was zu einer weiteren Steigerung der Investkosten führt. Zudem wird die aktivierte Nickeloberfläche während der Elektrolyse passiviert und muss häufig regeneriert werden, was zu einer Steigerung der Prozesskosten führt.

Propiolsaure kann außerdem durch Oxidation von Propargylalkohol mit Cr(VI)-Oxid in Schwefelsäure erhalten werden. Hier sind gute Ausbeuten erzielbar, aber große Mengen an toxischen und umweltgefährdenden Schwermetallsalzen zu entsorgen. Die analoge Umsetzung von 2-Butin-1,4-diol führt nur mit 23 % zu Acetylendicarbonsäure (Heilbron, Jones und Sondheimer, J. Chem. Soc. 1949, 606).

Als nicht-oxidatives Herstellverfahren von Propiolsäure und Acetylendicarbonsäure ist die Umsetzung von Metall-acetyliden mit CO₂ bekannt. Dies erfordert aber den Einsatz teurer Metallbasen und ist technisch wegen des Einsatzes von Acetylen nicht ohne Gefahr. Die Ausbeuten dieses Verfahrens liegen im Falle von Propiolsäure ebenfalls nur bei 50%.

Bei einem weiteren Verfahren zur Darstellung von Acetylendicarbonsäure wird Fumarsäure mit Brom zunächst in meso-Dibrombernsteinsäure überführt, diese isoliert und in einer weiteren Stufe enthalogeniert. Dieser Zweistufenprozess ist zeitaufwändig und umständlich (T.W. Abbott et al., Org. Synth Coll. Vol. II, 1943, 10).

Allgemeine Oxidationsverfahren von Alkoholen zu Carbonsäuren mit Hilfe von Nitroxylverbindungen als Katalysatoren sind aus dem Stand der Technik bekannt, insbesondere mit Hilfe von Nitroxylen wie TEMPO (2,2,6,6,-Tetramethylpiperidin-1-oxyl) und seinen Derivaten (Übersicht in A.E.J. de Nooy, A.C. Besemer und H.V. Bekkum, Synthesis 1996, 1153).
TEMPO-katalysierte Oxidationen von Alkoholen zu Carbonsäuren werden in Zweiphasensystemen, z.B. Methylenchlorid / Wasser, sowie in Gegenwart von Phasentransferkatalysatoren durchgeführt (G. Grigoropolulou et al., Chem. Commun. 2001, 547-548, P.L. Anelli, C. Biffi, F. Montanari und S. Quici, J. Org. Chem. 1987, 52, 2559). Als stöchiometrisches Oxidationsmittel wird dabei vorwiegend Bleichlauge (Hypochloritlösung) eingesetzt.

Bei der üblichen Durchführung dieser Synthesen in zweiphasigen Systemen wird das in der Wasserphase gelöste Oxidationsmittel, das auf einen pH-Bereich von 8.5-9 eingestellt ist, zu einer vorgelegten organischen Phase, die den zu oxidierenden Alkohol, den Phasentransferkatalysator und die Nitroxylverbindung enthält, in einem Batch-Verfahren zugesetzt.

Im Stand der Technik wird beschrieben, dass derartige Oxidationsverfahren unter Verwendung von Bleichlauge und Nitroxylverbindungen für die Oxidation ungesättigter Alkohole generell als ungeeignet zu betrachten sind (vergleiche hierzu insbesondere P.L. Anelli, C. Biffi, F. Montanari und S. Quici, J. Org. Chem. 1987, 52, 2559; P.L. Anelli, F. Montanari und S. Quici, Org. Synth., 1990, 69, 212). ,
So liefert die Umsetzung eines Alkinalkohols ohne terminale Alkingruppe (3-Phenylpropinol) mit Bleichlauge und TEMPO nach dem im Stand der Technik bekannten Verfahren nur unakzeptabel geringe Ausbeuten von 5 bis maximal 20 mol% der Alkinsäure (M. Zhao et al., J. Org. Chem. 1999, 64, 2564; WO 99/52849).

Die Oxidation von Alkinolen mit terminaler Alkingruppe mit Bleichlauge und TEMPO bei pH > 7 wurde bislang nicht beschrieben.

Eine mögliche Ursache ist die literaturbekannte Empfindlichkeit terminaler Alkingruppen gegenüber Bleichlauge. Die CH-Gruppen terminaler Alkine werden beispielsweise durch Bleichlauge leicht in Chloralkine überführt, die besonders im Alkalischen labil sind und zur Zersetzung neigen (Straus et al., Ber. Dtsch. Chem. Ges. 1930, 1868). Dies gilt insbesondere bei alkalischen Reaktionsbedingungen, da dabei die acide terminale Acetyleneinheit besonders leicht halogeniert wird. Die dabei entstehenden 3-Halogenopropiolate sind zudem zersetzliche Verbindungen, die zur Explosion neigen.

Es bestand daher die Aufgabe, ein Verfahren zur Herstellung von Alkinsäuren durch die Oxidation von Alkinalkoholen bereitzustellen, dass die aus dem Stand der Technik bekannten Nachteile vermeidet.

Überraschend wurde nun gefunden, dass sich die Nachteile der nach dem Stand der Technik bekannten Verfahren vermeiden lassen, indem der zu oxidierende Alkinalkohol in Gegenwart von Nitroxylverbindungen bei pH > 7 nicht wie in den aus dem Stand der Technik bekannten Verfahren vollständig vorgelegt wird, sondern wie das Oxidationsmittel fortlaufend der Reaktionsmischung zugegeben wird.

Gegenstand der Erfindung ist ein Verfahren zur Herstellung von Alkincarbonsäuren, gekennzeichnet durch die Oxidation eines Alkinalkohols mit einem Hypohalogenit in Gegenwart einer Nitroxylverbindung bei einem pH-Wert größer 7 unter fortlaufender Zugabe des Alkinalkohols und des Hypohalogenits zur Reaktionsmischung.

Die fortlaufende Zugabe des Alkinalkohols und des Hypohalogenits zur Reaktionsmischung nach dem erfindungsgemäßen Verfahren kann dabei kontinuierlich oder diskontinuierlich erfolgen.

Bei der kontinuierlichen Zugabe wird ständig eine mehr oder weniger große Menge, in Abhängigkeit von verschiedenen zu überwachenden Reaktionsparametern, an Alkinalkohol und Hypohalogenit zudosiert.

Bei der diskontinuierlichen Zugabe wird schubweise Alkinalkohol und Hypohalogenit, in Abhängigkeit von verschiedenen zu überwachenden Reaktionsparametern, zudosiert.

Die fortlaufende Zugabe des Alkinalkohols und des Hypohalogenits zur Reaktionsmischung kann sowohl bei der kontinuierlichen, wie auch der diskontinuierlichen Methode parallel oder alternierend erfolgen.

Bei der alternierenden Dosierung werden Alkinalkohol und Hypohalogenit nicht zeitgleich und/oder nicht in gleichen molaren Mengen innerhalb eines Zeitintervalls zudosiert.

Bei der parallelen Dosierung werden Alkinalkohol und Hypohalogenit immer zeitgleich zudosiert (Paralleldosierung).

Die zu überwachenden Reaktionsparameter bei der fortlaufenden Zugabe des Alkinalkohols und des Hypohalogenits zur Reaktionsmischung betreffen dabei insbesondere den pH-Wert der Reaktionsmischung sowie deren Temperatur.

In einer besonders bevorzugten Ausführungsform der Erfindung erfolgt die Zugabe von Alkinalkohol und Hypohalogenit fortlaufend und parallel.

In einer bevorzugten Ausführungsform des erfindungsgemäßen Verfahrens kann eine Vorlage verwendet werden, die Wasser, eines oder mehrere inerte organische Lösemittel, Säuren, Basen oder Puffergemische, Teilmengen oder die Gesamtmengen der eingesetzten Nitroxylverbindungen und Phasentransferkatalysatoren, sowie Teilmengen des eingesetzten Alkinalkohols oder Teilmengen des eingesetzten Oxidationsmittels enthalten kann.

Die Umsetzung erfolgt dann durch weitere fortlaufende Zugabe des Alkinalkohols und des Hypohalogenits zur Vorlage.

Die Verwendung einer Vorlage empfiehlt sich insbesondere für den Beginn einer kontinuierlichen Umsetzung.

Ferner wurde überraschend gefunden, dass Alkinalkohole auch ohne den Einsatz von Phasentransferkatalysatoren zu Alkincarbonsäuren oxidiert werden können.

Die Oxidation ist überraschender Weise sogar in einem wässrigeinphasigen System möglich.

Ferner wurde überraschend gefunden, dass sich nach dem erfindungsgemaßen Verfahren auch Alkinalkohole mit terminalen Alkingruppen bei pH > 7 in ausgezeichneten Ausbeuten oxidiert werden.

Im allgemeinen sind die zu oxidierenden Alkinalkohole (Alkinole) Verbindungen, die mindestens eine einbindige Gruppierung der Formel -CH2-OH und mindestens eine zweibindige Gruppierung der Formel -C=C- enthalten.

Bevorzugt handelt es sich bei den zu oxidierenden Alkinalkoholen um lineare oder verzweigte primäre Alkohole mit 3-30 C-Atomen, um zyklische Alkohole mit 8-30 C-Atomen oder um durch einen aromatischen Rest substituierte Alkohole mit 6-30 C-Atomen, die mindestens eine Gruppe der Formel -C≡C- enthalten,
wobei ein Wasserstoff oder mehrere Wasserstoffe unabhängig voneinander durch F, Cl, Br, I, NO₂ ,ONO, CN, NC oder SCN ersetzt sein können,
oder wobei eine CH₂-Gruppe oder mehrere CH₂-Gruppen unabhängig voneinander durch O, NH, C=O, CO₂, S, S=O, SO₂, P=O oder PO₂ ersetzt sein können,
oder eine CH-Gruppe oder mehrere CH-Gruppen unabhängig voneinander durch N, B oder P ersetzt sein können, oder quartäre Kohlenstoffe durch Si, Sn oder Pb ersetzt sein können.

Besonders bevorzugt sind Alkinalkohole R¹-C≡C-CH₂OH, R¹-C≡C-CH₂-CH₂OH oder R¹-C≡C-CH₂-CH₂-CH₂OH, R¹-O-CR²R³-C≡C-CH₂OH, R¹-O-CR²R³-C≡C-CH₂-CH₂OH oder R¹-O-CR²R³-C≡C-CH₂-CH₂-CH₂OH
mit R¹ in der Bedeutung H, Methyl, Ethyl oder eines linearen oder verzweigten C₃-C₁₂-Rests, insbesondere eines n-Propyl-, Isopropyl-, 1-oder-2-n-Butyl-, 2-Methylpropyl-, 1-, 2- oder 3-n-Pentyl-, 2- oder 3-Methyl-1-butyl-, 1,2-Dimethylpropyl-, tert-butyl-, neopentyl- oder tert-pentylrests,
oder eines gesättigten oder ungesättigten cyclischen C₃-C₁₂-Rests, insbesondere eines Cyclopropyl-, Cyclobutyl-, Cyclopentyl-, Cyclopentenyl-, Methylcyclopentyl-, Methylcyclopentenyl , Cyclohexyl-, Cyclohexenyl-, Methylcyclohexyl-, Methylcyclohexenyl-, Cycloheptyl-, Cyclooctyl-, Cyclododecyl- oder Decalinylrests
oder eines C₆-C₁₂-Aryl- oder Aralkylrests, insbesondere eines Phenyl-, Benzyl-, Phenethyl-, Naphthyl-, Biphenylyl-, Anthryl-, Phenanthryl-, Azulenyl-, Anthrachinonyl-, 2-, 3- oder 4-Methylphenyl-, 2,3-, 2,4 oder 2,5-Dimethylphenyl- oder Mesitylylrests,
oder eines C₆-C₁₂-Heteroaryl- oder Heteroaralkylrests, insbesondere eines Furyl-, Pyrrolyl-, Thienyl-, Benzofuranyl-, Isobenzofuranyl-, Benzothiyl-, Isobenzothienyl-, Indolyl-, Isoindolyl-, Indolizinyl-, Pyrazolyl-, Imidazolyl-, Oxazolyl-, Thiazolyl-, Isoxazolyl-, Isothiazolyl-, Indazolyl-, Carbazolyl-, Benzotriazolyl-, Purinyl-, Pyridyl-, Pyridazinyl-, Pyrimidyl-, Pyrazinyl-, Chinolinyl-, Isochinolinyl-, Chinoxalinyl-, Chinazolinyl-, Cinnolinyl-, Phenanthridinyl-, Acridinyl-, 1,10-Phenanthrolinyl-, Phenazinyl-, Phenothiazinyl- oder Phenoxazinylrests.
oder in der Bedeutung R⁴R⁵R⁶Si mit R⁴, R⁵ und R⁶ unabhängig voneinander in der Bedeutung C₁-C₁₂-Alkyl, insbesondere Methyl, Ethyl, n-Propyl, Isopropyl oder n-Butyl,
oder C₁-C₁₂-Oxyalkyl, insbesondere Methoxy, Ethoxy, n-Propoxy, Isopropoxy oder Butoxy,
C₆-C₁₂-Aryl oder C₇-C₁₂-Aralkyl, insbesondere Phenyl oder Benzyl,
und R² und R³ unabhängig voneinander in der Bedeutung H, C₁-C₁₂-Alkyl, insbesondere Methyl, Ethyl, n-Propyl oder n-Butyl, C₆-C₁₂-Aryl oder C₇-C₁₂-Aralkyl, insbesondere Phenyl, 2-, 3- oder 4-Methylphenyl oder Benzyl
und Alkinole R⁷-CO-C≡C-CH₂OH, R⁷-CO-C≡C-CH₂-CH₂OH oder R⁷-CO-C≡C-CH₂-CH₂-CH₂OH mit R⁷ in der Bedeutung Methyl, Ethyl oder eines linearen oder verzweigten C₃-C₁₂-Rests, insbesondere eines n-Propyl-, Isopropyl-, 1-oder-2-n-Butyl-, 2-Methylpropyl-, 1-, 2- oder 3-n-Pentyl-, 2- oder 3-Methyl-1-butyl-, 1,2-Dimethylpropyl-, tert-butyl-, neopentyl- oder tert-pentylrests,
oder eines gesättigten oder ungesättigten cyclischen C₃-C₁₂-Rests, insbesondere eines Cyclopropyl-, Cyclobutyl-, Cyclopentyl-, Cyclopentenyl-, Methylcyclopentyl-, Methylcyclopentenyl-, Cyclohexyl-, Cyclohexenyl-, Methylcyclohexyl-, Methylcyclohexenyl-, Cycloheptyl-, Cyclooctyl-, Cyclododecyl- oder Decalinylrests
oder eines C₆-C₁₂-Aryl- oder Aralkylrests, insbesondere eines Phenyl-, Benzyl-, Phenethyl-, Naphthyl-, Biphenylyl-, Anthryl-, Phenanthryl-, Azulenyl-, Anthrachinonyl-, 2-, 3- oder 4-Methylphenyl-, 2,3-, 2,4 oder 2,5-Dimethylphenyl- oder Mesitylylrests,
oder eines C₆-C₁₂-Heteroaryl- oder Heteroaralkylrests, insbesondere eines Furyl-, Pyrrolyl-, Thienyl-, Benzofuranyl-, Isobenzofuranyl-, Benzothiyl-, Isobenzothienyl-, Indolyl-, Isoindolyl-, Indolizinyl-, Pyrazolyl-, Imidazolyl-, Oxazolyl-, Thiazolyl-, Isoxazolyl-, Isothiazolyl-, Indazolyl-, Carbazolyl-, Benzotriazolyl-, Purinyl-, Pyridyl-, Pyridazinyl-, Pyrimidyl-, Pyrazinyl-, Chinolinyl-, Isochinolinyl-, Chinoxalinyl-, Chinazolinyl-, Cinnolinyl-, Phenanthridinyl-, Acridinyl-, 1,10-Phenanthrolinyl-, Phenazinyl-, Phenothiazinyl- oder Phenoxazinylrests.
sowie Cl-CH₂-C≡C-CH₂OH und HO-CH₂-C≡C-CH₂OH
Ganz besonders bevorzugt sind 2-Propin-1-ol, But-3-in-1-ol, But-2-in-1-ol, Pent-4-in-1,2-diol, 2-Butin-1,4-diol, 4-Chlor-2-butin-1-ol, 4-Acetoxy-2-butin-1-ol, 4-t-Butyldimethylsiloxy-2-butin-1-ol, 3-Phenyl-2-propin-1-ol, 3-Trimethylsilyl-2-propin-1-ol, 3-t-Butyldimethylsilyl-2-propin-1-ol.

Insbesondere eignen sich 2-Propin-1-ol, 4-Chlor-2-butin-1-ol oder 2-Butin-1,4-diol, ganz besonders bevorzugt 2-Propin-1-ol oder 2-Butin-1,4-diol.

Bei der als Oxidationskatalysator eingesetzten Nitroxylverbindung handelt es sich im allgemeinen um eine Di-tert.-alkylnitroxylverbindung.

Bevorzugt handelt es sich um eine Nitroxylverbindung der allgemeinen Formel I 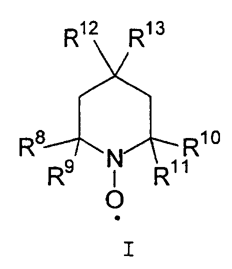 worin die Reste R⁸, R⁹, R¹⁰ und R¹¹ unabhängig voneinander C₁-C₁₂-Alkyl oder C₂-C₁₂-Alkenyl oder C₆-C₁₂-Aryl oder Aralkyl bedeuten,
und die Reste R¹² und R¹³ unabhängig voneinander folgende Bedeutung haben: Wasserstoff, OH, CN, Halogen,
linear oder verzweigt, gesättigt oder ungesättigt C₁-C₂₀-Alkyl-, C₆-C₂₀-Aryl, C₆-C₂₀-Hetaryl oder C₆-C₂₀-Aralkyl, OR¹⁴, O-COR¹⁴, O-COOR¹⁴, OCONHR¹⁴, COOH, COR¹⁴, COOR¹⁴, CONHR¹⁴
wobei R¹⁴ die Bedeutung eines linearen oder verzweigten, gesättigten oder ungesättigten C₁-C₂₀-Alkylrestes, oder die Bedeutung eines C₆-C₂₀-Aryl, C₃-C₂₀-Hetaryl oder C₆-C₂₀-Aralkylrests hat,
-(O-CH₂-CH₂)ₙ-OR¹⁵, -(O-C₃H₆)ₙ-OR¹⁵, -(O-(CH₂)₄)ₙ-OR¹⁵, -O-CH₂-CHOH-CH₂-(O-CH₂-CH₂-)ₙ-OR¹⁵
mit R¹⁵ in der Bedeutung Wasserstoff, C₁-C₂₀-Alkyl, C₆-C₂₀-Aralkyl, mit n = 1 bis 100 , oder CH₂-CHOH-CH₃ oder CH₂-CHOH-CH₂-CH₃,
NR¹⁶R¹⁷, NHCOR¹⁶, NHCOOR¹⁶, NHCONHR¹⁶,
mit R¹⁶ und R¹⁷ unabhängig voneinander in der Bedeutung eines linearen oder verzweigten, gesättigten oder ungesättigten C₁-C₂₀-Alkylrestes, eines C₆-C₁₂-Cycloalkylrests, oder eines C₆-C₂₀-Aryl , C₃-C₂₀ Hetaryl oder C₆-C₂₀-Aralkylrests,
wobei die Reste R¹² und R¹³ auch zu einem Ring verknüpft sein können,
und wobei die Reste R¹² und R¹³ ihrerseits auch mit COOH, OH, SO₃H, CN, Halogen, prim., sek., tert. Amino oder quart. Ammonium substituiert sein können,
oder die Reste R¹² und R¹³ zusammen auch die Bedeutung =O, =NR¹⁸, =N-OR¹⁸, =N-N=CR¹⁸R¹⁹ mit R¹⁸ und R¹⁹ unabhängig in der Bedeutung Wasserstoff, C₁-C₂₀-Alkyl oder C₆-C₂₀-Aralkyl haben können.

Weiterhin bevorzugt handelt es sich bei der Nitroxylverbindung um zwei Moleküle der Formel I, die über eine Brücke =N-N= in 4-Position verknüpft sind.

Weiterhin bevorzugt handelt es sich bei der Nitroxylverbindung um zwei oder mehrere Moleküle der allgemeinen Formel I, die über einen der beiden Reste R¹² oder R¹³ miteinander verbunden sind. Dabei eignet sich als verknüpfender Rest besonders bevorzugt um O-Alkyl-O, O-CH₂-Aryl-CH₂-O, oder eine Brücke der allgemeinen Formel (O-(CH₂)ₙ-O)ₘ mit n = 2 bis 4 und m = 2 bis 50, insbesondere m = 2 bis 20.

In einer weiteren Ausführungsform handelt es sich bei der Nitroxylverbindung um eine polymere Struktur enthaltend Verbindungen der Formel I, die über die Reste R¹¹ oder R¹² oder R¹¹ und R¹² verknüpft ist.

Dem Fachmann sind eine Vielzahl solcher Verbindungen aus dem Stand der Technik bekannt (EP 1103537, Cirriminna et al., Chem. Commun. 2000, 1441; Bolm et al., Chem. Commun. 1999, 1795; Bobbitt et al., Chem. Commun. 1996, 2745, Miyazawa und Endo, J. Molec. Catal. 49, 1988, L31; M. J. Verhoef et al. in "Studies in Surface Science and Catalysis", Vol. 125, S. 465 ff; D. Brunel et al. in "Studies in Surface Science and Catalysis", Vol. 125, S. 237 ff; Miyazawa und Endo, J. Polymer Sci., Polym. Chem. Ed. 23, 1985, 1527 und 2487; T. Osa, Chem. Lett. 1988, 1423).

Insbesondere eignen sich PIPO (polyamine immobilised piperidinyl oxyl), SiO₂-geträgertes TEMPO, Polystyrol- und Polyacrylsäure-geträgertes TEMPO.

Als Nitroxylverbindung besonders bevorzugt sind Verbindung der allgemeinen Formel I mit R⁸, R⁹, R¹⁰ und R¹¹ in der Bedeutung CH₃
und R¹² und R¹³ unabhängig voneinander in der Bedeutung Wasserstoff, OH, OR¹⁴, O-COR¹⁴, O-COOR¹⁴, OCONHR¹⁴,
wobei R¹⁴ die Bedeutung eines linearen oder verzweigten, gesättigten oder ungesättigten C₁-C₂₀-Alkylrestes, oder die Bedeutung eines C₆-C₂₀-Aryl oder C₆-C₂₀-Aralkylrests hat,
-(O-CH₂-CH₂)ₙ-OR¹⁵, -(O-C₃H₆)ₙ-OR¹⁵, -(O-(CH₂)₄) ₙ-OR¹⁵, -O-CH₂-CHOH-CH₂-(O-CH₂-CH₂-)ₙ-OR¹⁵
mit R¹⁵ in der Bedeutung Wasserstoff, C₁-C₁₀-Alkyl oder C₆-C₁₀-Aralkyl, mit n = 1 bis 100, oder CH₂-CHOH-CH₃ oder CH₂-CHOH-CH₂-CH₃,
NR¹⁶R¹⁷, NHCOR¹⁷, NHCOOR¹⁷, NHCONHR¹⁷,
mit R¹⁶ und R¹⁷ unabhängig voneinander in der Bedeutung Wasserstoff, eines linearen oder verzweigten, gesättigten oder ungesättigten C₁-C₂₀-Alkylrestes, eines C₆-C₁₂-Cycloalkylrests oder eines C₆-C₂₀-Aryl oder C₆-C₂₀-Aralkylrests.

Als Nitroxylverbindung besonders bevorzugt sind weiterhin Verbindungen der allgemeinen Formel I mit R⁸, R⁹, R¹⁰ und R¹¹ in der Bedeutung CH₃
worin R¹² und R¹³ gemeinsam Ketal-Gruppierungen der Formeln O-CHR²⁰CHR²¹-O oder O-CH₂CR²²R²³-CH₂-O mit R²⁰ , R²¹, R²² und R²³ unabhängig voneinander in der Bedeutung Wasserstoff oder C₁-C₃-Alkyl bilden,
oder worin die Reste R¹² und R¹³ zusammen die Bedeutung =O haben.

Als Nitroxylverbindung insbesondere bevorzugt sind eine Verbindung der allgemeinen Formel I mit R⁸, R⁹, R¹⁰ und R¹¹ in der Bedeutung CH₃
worin R¹² die Bedeutung Wasserstoff und R¹³ die Bedeutung Wasserstoff, OH, OR¹⁴, O-COR¹⁴,
wobei R¹⁴ die Bedeutung eines linearen oder verzweigten gesättigten C₁-C₁₂-Alkylrestes, oder die Bedeutung eines Aryl- oder Benzylrests hat,
-(O-CH₂-CH₂)ₙ-OR¹⁵, -(O-C₃H₆)ₙ-OR¹⁵, -(O-(CH₂)₄)ₙ-OR¹⁵, -O-CH₂-CHOH-CH₂-(O-CH₂-CH₂-)n-OR¹⁵ mit n = 1 bis 50
und R¹⁵ in der Bedeutung Wasserstoff oder CH₂-CHOH-CH₃ oder CH₂-CHOH-CH₂-CH₃

NR¹⁶R¹⁷, NHCOR¹⁷, mit R¹⁶ und R¹⁷ unabhängig voneinander in der Bedeutung eines linearen oder verzweigten gesättigten C₁-C₁₂-Alkylrestes, oder eines Aryl- oder Benzylrests.

Beispiele für besonders bevorzugt einsetzbare Nitroxylverbindungen sind TEMPO, 4-Hydroxy-TEMPO, 4-Oxo-TEMPO, 4-Amino-TEMPO, 4-Acetamido-TEMPO, 4-Benzoyloxy-TEMPO, 4-Acetoxy-TEMPO und PIPO.

Ganz besonders bevorzugt ist 4-Hydroxy-TEMPO.

Die Nitroxylverbindung wird im allgemeinen in Mengen von 0,01 bis 50 Mol %, bevorzugt in Mengen von 0,1 bis 20 Mol %, besonders bevorzugt in Mengen von 1 bis 10 Mol % bezogen auf die Menge an zu oxidierendem Alkinalkohol eingesetzt.

Sie kann in der den Alkinalkohol enthaltenden Reaktionskomponente oder in der wässrigen Phase gelöst oder in geträgerter Form als eigenständige Phase eingesetzt werden. Die Nitroxylverbindung kann vollständig vorgelegt werden oder kontinuierlich zur Reaktionsmischung hinzugegeben werden, ggf. auch in Form einer zusätzlichen flüssigen Dosierung.

Als Oxidationsmittel wird bevorzugt eine Verbindung eingesetzt, ausgewählt aus der Gruppe der Hypohalogenite, insbesondere Hypochlorit, Hypobromit und Hypoiodit oder deren Gemische. Besonders bevorzugtes Oxidationsmittel ist Hypochlorit. Als Gegenionen sind Wasserstoff, Natrium, Kalium, Calcium oder Tetraalkylammonium bevorzugt, besonders bevorzugt sind Natrium und Calcium.

Dem Fachmann sind aus dem Stand der Technik geeignete Hypohalogenite und Hypohalogenit-Zubereitungen bekannt (Ullmann Encyclopedia, 6^{th} Edition, 2002 electronic release; "Chlorine oxides and Chlorine oxygen acids 2. - 4.").

In einer besonders bevorzugten Ausführungsform werden technische Hypohalogenitlösungen und -suspensionen, insbesondere technische Hypochloritlösungen eingesetzt.
Das verwendete Oxidationsmittel kann auch in situ, insbesondere elektrochemisch, durch Hydrolyse, insbesondere durch Hydrolyse von N-Chlorverbindungen, oder durch Redoxreaktionen, wie bei Hypochlorit- oder Hypobromitlösungen durch Disproportionierung von Chlor bzw. Brom in alkalisch-wässriger Lösung, oder durch die Redoxreaktion zwischen Hypochlorit und Bromid, die zur Bildung von Hypobromit führt, erzeugt werden.

Die verwendeten Oxidationsmittel, insbesondere Hypochlorit und Hypobromit werden vorzugsweise in Konzentrationen von 0,1 M bis zu ihrer jeweiligen Sättigungskonzentration als wässrige Lösungen eingesetzt.

Der pH-Wert von wässrigen Lösungen oder Suspensionen der Oxidationsmittel liegen im allgemeinen zwischen 7 und 14. Eine besondere Einstellung des pH des Oxidationsmittel ist jedoch nicht erforderlich, so dass vorteilhafterweise auf eine vorherige pH-Einstellung des Oxidationsmittel verzichtet werden kann.

Der pH-Wert der wässrigen Phase der Reaktionsmischung liegt nach dem erfindungsgemäßen Verfahren bei einem pH-Wert größer 7, bevorzugt zwischen pH 7 und 14, besonders bevorzugt zwischen pH 7 und 11, insbesondere zwischen pH 8 und 10.

Bevorzugt werden 2 bis 5 mol-Äquivalente des Hypohalogenits bezogen auf die Anzahl zu oxidierender funktionaler Gruppen, insbesondere 2 bis 3 mol-Äquivalente des Hypohalogenits bezogen auf die Anzahl zu oxidierender funktionaler Gruppen verwendet.

Die Einstellung des gewünschten pH-Werts der Reaktionsmischung erfolgt im allgemeinen durch Zugabe einer Base, bevorzugt Natriumhydroxid, Kaliumhydroxid, Calciumhydroxid, Calciumcarbonat, Natriumcarbonat, besonders bevorzugt Natriumhydroxid und Calciumcarbonat.

Die Einstellung des gewünschten pH-Werts der Reaktionsmischung kann ferner durch Zugabe eines Puffers, bevorzugt durch Zugabe eines Carbonat- oder Phosphatpuffers erfolgen. Aus der Gruppe der Carbonatpuffer eignen sich besonders Natriumcarbonat/Natriumhydrogencarbonat oder Calciumcarbonat/Calciumhydrogencarbonat, insbesondere Calciumcarbonat/Calciumhydrogencarbonat. Aus der Gruppe der Phosphatpuffer eignen sich besonders Natriumsalze oder Kaliumsalze der Phosphorsäure.
Weitere mögliche Zusätze sind Salze, z.B. Alkali-, Erdalkalioder Ammoniumhalogenide, -carbonate oder -sulfate.

Die Reaktionstemperatur beträgt im allgemeinen -10 bis +80°C, bevorzugt -5 bis +40°C, besonders bevorzugt -5 bis +20°C.

Das erfindungsgemäße Verfahren wird bevorzugt bei Normaldruck durchgeführt.

Generell können in dem erfindungsgemäßen Verfahren die an der Reaktion beteiligten Komponenten in einer Phase oder auf mehrere Phasen verteilt umgesetzt werden.

In einer möglichen Ausführungsform wird das erfindungsgemäße Verfahren in einer flüssigen Phase, die Wasser und ein oder mehrere mit Wasser mischbare Lösungsmittel enthalten (Cosolventien), durchgeführt.

Bei einer Ausführungsform der einphasigen Reaktion wird der zu oxidierende Alkinalkohol in reiner Form oder verdünnt mit Wasser oder mit einem oder mehreren inerten mit Wasser mischbaren Solventien als Reaktionskomponente 1 und das Oxidationsmittel als Reaktionskomponente 2 dosiert.

Die inerten mit Wasser mischbaren Solventien werden bevorzugt ausgewählt aus der Gruppe der Ether, insbesondere THF und 1,4-Dioxan, der Nitrile, insbesondere Acetonitril oder Alkohole wie z. B. tert-Butanol, Isopropanol oder DMF, DMSO.

Der zu oxidierende Alkinalkohol kann in Konzentrationen zwischen 0,1 und 100 Gew. %, bevorzugt zwischen 20 und 100 Gew. % bezogen auf die Reaktionskomponente 1 eingesetzt werden.

In einer weiteren möglichen Ausführungsform des erfindungsgemäßen Verfahren wird die Reaktionen in Mehrphasensystemen durchgeführt.

Bevorzugt wird dabei mindestens eine wässrige Phase und eine organische Phase verwendet.

In einer besonders bevorzugten Ausführungsform wird der Alkinalkohol gegebenenfalls in reiner Form oder in einem oder mehreren Lösungsmitteln gelöst, als Reaktionskomponente 1 eingesetzt. Bevorzugt werden mit Wasser nicht mischbare organische Lösungsmittel verwendet. Die entstehende Phasenseparation kann dabei auch ursächlich auf ein nicht mit Wasser mischbaren Alkinalkohol als Edukt zurückgehen.

Die wässrige Phase als Reaktionskomponente 2 enthält das Oxidationsmittel.

Bevorzugte organische Lösungsmittel zur Durchführung des Verfahrens in einem Mehrphasensystem sind ein oder mehrere Lösungsmittel ausgewählt aus der Gruppe der Ether, insbesondere THF, Methyl-t-Butylether, Dimethoxymethan und Diethylether, Chlorkohlenwasserstoffe wie z.B. Methylenchlorid, Ester wie z.B. Essigsäureethylester, Alkohole wie z.B. tert-Butanol, Kohlenwasserstoffe wie z.B. Toluol, Cyclohexan, Heptan sowie Dimethylsulfoxid (DMSO), Acetonitril.

Der zu oxidierende Alkohol kann in Konzentrationen zwischen 0,1 und 100 Gew. % bezogen auf die Reaktionskomponente 1, bevorzugt zwischen 20 und 100 Gew. % eingesetzt werden.

In einer weiteren bevorzugten Ausführungsform der Reaktion in Mehrphasensystemen wird das erfindungsgemäße Verfahren in Gegenwart eines oder mehrerer Phasentransferkatalysatoren durchgeführt. Geeignete Phasentransferkatalysatoren sind dem Fachmann bekannt (siehe z. B. "Phase transfer catalysis", ULLMANN'S ENCYCLOPEDIA OF INDUSTRIAL CHEMISTRY, 6th Edition electronic release, 2002). Bevorzugte Phasentransferkatalysatoren sind quartäre Ammoniumsalze, wie z.B. Tetrabutylammoniumchlorid, Tetrabutylammoniumbromid, Tetrabutylammoniumsulfat, Methyltrioctylammoniumchlorid, Methyltributylammoniumchlorid, Benzyltrimethylammoniumchlorid, Triethylbenzylammoniumchlorid, und Kronenether wie z.B. 18-Krone-6.
Der Phasentransferkatalysator kann in Mengen zwischen 0.1 und 10 Mol.% bezogen auf den zu oxidierenden Alkinalkohol eingesetzt werden, wenn das erfindungsgemäße Verfahren in Gegenwart einer wässrigen sowie mindestens einer organischen Phase durchgeführt wird. Der Phasentransferkatalysator kann sich in der Vorlage befinden, kann aber auch der Reaktionskomponente 1 oder 2 zugesetzt werden.

Das erfindungsgemäße Verfahren kann im Batch-Betrieb oder in Form einer kontinuierlichen Umsetzung durchgeführt werden.

In einer bevorzugten Ausführungsform des erfindungsgemäßen Verfahres werden die an der Reaktion beteiligten Phasen kontinuierlich umgesetzt.

Dabei werden Alkinalkohol und Hypohalogenit in Form einer erfindungsgemäßen fortlaufenden Zugabe zugeführt und zugleich die entstehende Reaktionslösung kontinuierlich aus dem Reaktor abgezogen.

Die Nitroxylverbindung wird dabei ebenfalls kontinuierlich zugeführt oder in Form einer stationären Phase eingesetzt. Die Einhaltung des für die Reaktion günstigen pH-Bereiches kann durch eine kontinuierliche Dosierung erfolgen, über die Basen oder Säuren der Reaktionsmischung so zugeführt wird, dass in der Reaktionsmischung ein konstanter pH > 7 vorliegt.

Die für eine kontinuierliche Umsetzung geeigneten kontinuierlichen Reaktoren sind dem Fachmann bekannt. Eine Übersicht über die wichtigsten Ausführungsformen findet sich beispielsweise in "Ullmann's Encyclopedia of Industrial Chemistry", Vol. B4.
Bevorzugte Ausführungsformen für ein kontinuierlich durchgeführtes Verfahren sind kontinuierlich betriebene Rohrreaktoren, kontinuierlich betriebene Loop-Reaktoren, kontinuierlich betriebene Rührkessel oder Rührkesselkaskaden oder ein mit Hilfe von Kreiselpumpen durchgeführtes Verfahren.

Bei einer kontinuierlichen Durchführung des erfindungsgemäßen Verfahrens wird eine Verweilzeit zwischen 0.1 sec bis 10 h eingestellt, bevorzugt zwischen 1 sec und 1 h, ganz besonders bevorzugt zwischen 1 sec und 20 min.

Wird das beschriebene Verfahren als kontinuierlicher Prozess ausgeführt, erlaubt dies als zusätzlichen Vorteil eine effiziente Wärmeabfuhr aus dem stark exothermen Reaktionsprozess.

Das relative Verhältnis der Volumenströme der beiden Reaktionskomponenten kann konstant gehalten werden, kann aber auch in Abhängigkeit von der in der Reaktionsmischung sich einstellenden Gehalte an noch nicht umgesetztem Alkinakohol, Alkinsäure und Oxidationsmittel verändert werden.

Die Dosierungsgeschwindigkeit beider Reaktionskomponenten erfolgt bevorzugt so, dass die Reaktionsmischung unter Berücksichtigung der verfügbaren Kühlleistung im bevorzugten Temperatur-Bereich bleibt.

Die Vorteile des erfindungsgemäßen Verfahrens liegt in der Bereitstellung eines technisch einfach durchführbaren Verfahrens zur Oxidation von Alkinalkoholen zu Alkincarbonsäuren unter Verwendung des kostengünstigen Oxidationsmittels Hypohalogenit, das die aus dem Stand der Technik bekannten Probleme löst.

Insbesondere durch die Maßnahmenkombination der Kontrolle des pH-Werts der wässrigen Phase der Reaktionsmischung und der fortlaufenden Zugabe der beiden Reaktanden Hypohalogenit und Alkinalkohol zur Reaktionsmischung können hohe Ausbeuten an Alkincarbonsäuren erhalten werden.
Mit dem erfindungsgemäßen Verfahren werden dabei unter Verwendung kostengünstiger und technisch leicht einsetzbarer Bleichlauge (wie z.B. Natriumhypochlorit) selbst gut wasserlösliche Alkinole und Alkinole mit terminalen Alkingruppen in technisch einfacher Weise und herausragender Ausbeute zu den entsprechenden Alkincarbonsäuren oxidiert.

Speziell Substrate mit terminalen Alkingruppen waren bislang durch die aus dem Stand der Technik bekannten oxidativen Verfahren nicht durch großtechnisch realisierbare und wirtschaftlich interessante Reaktionen zugänglich.

Darüber hinaus ist das erfindungsgemäße Verfahren für eine große Breite an Substraten mit terminalen und nicht terminalen Alkingruppen anwendbar.

So kann beispielsweise Propiolsäure mit den erfindungsgemäßen Verfahren aus Propargylalkohol in 75-90 % Ausbeute und Acetylendicarbonsäure aus Butindiol in 50-70 % Ausbeute erhalten werden.

Die bei der Umsetzung entstehenden Abwässer enthalten nur leicht zu entsorgende Salze wie NaCl und sind daher problemlos zu entsorgen. Ferner vermeidet das erfindungsgemäße Verfahren das Sicherheitsrisiko großer Vorlagen von Alkinolen, die bei Dosierungsfehlern des Oxidationsmittels Anlass zu unkontrollierten Reaktionen (wie z.B. zur Bildung der Chloralkinverbindungen) geben können.

Die aus dem Stand der Technik bekannten oxidativen Verfahren waren nur im Batch-Betrieb möglich und somit hinsichtlich der großtechnischen Realisierbarkeit und der Wirtschaftlichkeit weniger interessant.

Alle vorstehenden Symbole der vorstehenden Formeln weisen ihre Bedeutung jeweils unabhängig voneinander auf.

Die folgenden Beispiele dienen der weiteren Erläuterung der Erfindung.

### Vergleichsbeispiel 1 : Oxidation von 2-Propin-1-ol zu Propiolsäure mit vollständiger Vorlage des Alkinalkohols

19,6 g (350 mmol) 2-Propin-1-ol werden zusammen mit 3,0 g (17,4 mmol) 4-Hydroxy-TEMPO und 5,9 g (17,5 mmol) Tetrabutylammoniumhydrogensulfat in 132 ml Wasser und 132 ml CH₂Cl₂ gelost und auf 5°C gekühlt (Reaktionskomponente 1).
14,0 g (350 mmol) NaOH werden in 427 g 2,4 M (ca. 350 ml, 840 mmol) Natriumhypochloritlösung (technische Bleichlauge; pH 14) gelost und auf 5°C gekühlt (Reaktionskomponente 2). Reaktionskomponente 1 wird in einem Kolben mit mechanischer Rührung und Mantelkühlung vollständig vorgelegt. Reaktionskomponente 2 wird so zugegeben, dass die Innentemperatur nicht über 10 °C steigt. Der pH der Reaktionsmischung wird währenddessen durch Zusatz von 20 %iger Schwefelsäure zwischen pH 8 und pH 10 gehalten.
Nach vollständiger Zugabe wird noch 10 min bei 10°C nachgerührt. Eine Analyse der beiden Phasen zeigt, dass ca. 91% des eingesetzten Alkohols umgesetzt wurde. Die organische Phase enthält 1,3 Mol% Propiolsäurepropargylester. Die wässrige Phase enthält 46 Mol% Propiolsäure.

### Beispiel 1 : Oxidation von 2-Propin-1-ol zu Propiolsäure in einem Zweiphasensystem und in Gegenwart von Phasentransferkatalysator

19,6 g (350 mmol) 2-Propin-1-ol werden zusammen mit 3,0 g (17,4 mmol) 4-Hydroxy-TEMPO in 20 g CH₂Cl₂ gelöst (Reaktionskomponente 1).
14,0 g (350 mmol) NaOH werden in 436 g 2,4 M (ca. 357 ml, 857 mmol) Natriumhypochloritlösung (technische Bleichlauge; pH 14) gelost und auf 5°C gekühlt (Reaktionskomponente 2).
In einem Kolben mit mechanischer Rührung werden 5,9 g (17,4 mmol) Tetrabutylammoniumhydrogensulfat in 132 ml Wasser und 132 ml CH₂Cl₂ vorgelegt und auf 5°C gekühlt. Reaktionskomponente 1 und 2 werden parallel unter guter Rührung und Kühlung so zugegeben, dass die Innentemperatur nicht über 10 °C steigt. Der pH der Reaktionsmischung wird währenddessen durch Zusatz von 20 Gew%iger Schwefelsäure zwischen pH 8 und pH 10 gehalten.
Nach vollständiger Zugabe wird noch 10 min bei 10°C nachgerührt.
Nach Abtrennung der organischen Phase (enthält den Phasentransferkatalysator) wird die wässrige Phase mit Salzsäure (20 Gew.%ig) auf pH 0 eingestellt und 3-mal mit je 100 ml Ethylacetat extrahiert. Die wässrige Phase wird verworfen.
Die Ethylacetat-Phasen werden vereint und ergeben nach teilweiser destillativer Entfernung des Ethylacetats eine ca. 50%ige Lösung von Propiolsäure in Ethylacetat, die 19,5 g (278 mmol) Propiolsäure enthält (Ausbeute 79 Mol%).

### Beispiel 2 : Oxidation von 2-Propin-1-ol zu Propiolsäure in einem Zweiphasensystem ohne Phasentransferkatalysator

19,6 g (350 mmol) 2-Propin-1-ol werden zusammen mit 3,0 g (17,4 mmol) 4-Hydroxy-TEMPO in 20 g CH₂Cl₂ gelöst und auf 5°C gekühlt (Reaktionskomponente 1).
14,0 g (350 mmol) NaOH werden in 440 g 2,3 M (ca. 360 ml, 828 mmol) Natriumhypochloritlösung (technische Bleichlauge; pH 14) gelöst und auf 5°C gekühlt (Reaktionskomponente 2).
In einem Glaskolben mit mechanischem Rührer werden 132 ml Wasser und 132 ml CH₂Cl₂ vorgelegt und auf 5°C gekühlt. Unter intensiver Rührung und Kühlung werden Reaktionskomponente 1 und Reaktionskomponente 2 parallel so zugegeben, dass die Innentemperatur nicht über 10 °C steigt. Parallel wird durch fortlaufende Zugabe von 20 Gew%iger Schwefelsäure der pH-Wert der Reaktionsmischung zwischen 8 und 10 gehalten.

Nach vollständiger Zugabe der Reaktionskomponenten wird noch 10 min nachgeruhrt.
Nach Abtrennung der organischen Phase (enthält 4 Mol% Propiolsäurepropargylester) wird die wässrige Phase mit Salzsaure (20 Gew.%ig) auf pH 0 eingestellt und 3-mal mit je 100 ml Ethylacetat extrahiert. Die wässrige Phase wird verworfen. Die Ethylacetat-Phasen werden vereint und ergeben nach teilweiser destillativer Entfernung des Ethylacetats eine ca. 50%ige Lösung von Propiolsäure in Ethylacetat, die 18,3 g (261 mmol) Propiolsäure enthält (Ausbeute 75 Mol%).

### Beispiel 3 : Oxidation von 2-Propin-1-ol zu Propiolsäure in einem wässrigen einphasigen System

19,6 g (350 mmol) 2-Propin-1-ol werden zusammen mit 3,0 g (17,4 mmol) 4-Hydroxy-TEMPO in 20 ml Wasser gelöst (Reaktionskomponente 1).
14,0 g (350 mmol) NaOH werden in 440 g 2,4 M (ca. 360 ml, 864 mmol) Natriumhypochloritlösung (technische Bleichlauge; pH 14) gelöst und auf 5°C gekühlt (Reaktionskomponente 2).
In einem Glaskolben mit mechanischem Rührer werden 132 ml Wasser vorgelegt und auf 5°C gekühlt. Unter intensiver Rührung und Kühlung werden Reaktionskomponente 1 und Reaktionskomponente 2 parallel so zugegeben, dass die Innentemperatur nicht über 10 °C steigt. Parallel wird durch fortlaufende Zugabe von 20 Gew%iger Schwefelsäure der pH-Wert der Reaktionsmischung zwischen 8 und 10 gehalten.
Nach vollständiger Zugabe der Reaktionskomponenten wird noch 10 min nachgerührt und ggf. noch vorhandenes Hypochlorit mit Natriumhydrogensulfitlösung vernichtet.
Die Reaktionsmischung wird mit 150ml CH₂Cl₂ extrahiert. Nach Abtrennung der organischen Phase (enthält 0,9 Mol% Propiolsäurepropargylester) wird die wässrige Phase mit Salzsäure (20 Gew.%ig) auf pH 0 eingestellt und 3-mal mit je 100 ml Ethylacetat extrahiert. Die wässrige Phase wird verworfen. Die Ethylacetat-Phasen werden vereint und ergeben nach teilweiser destillativer Entfernung des Ethylacetats eine ca. 50%ige Lösung von Propiolsäure in Ethylacetat, die 20,3 g (290 mmol)Propiolsäure enthält (Ausbeute 83 Mol%). Diese Lösung kann direkt für die Darstellung von Propiolsäureethylester eingesetzt werden.
Zur Gewinnung reiner Propiolsäure wird diese Lösung mit 100 ml Toluol versetzt und dann Ethylacetat abdestilliert. Aus der verbleibenden Lösung wird dann die Propiolsäure durch Destillation isoliert.

### Beispiel 4: Kontinuierliche Oxidation von 2-Propin-1-ol zu Propiolsäure

408 g (7278 mmol) 2-Propin-1-ol werden mit 37.6 g (218 mmol) 4-Hydroxy-TEMPO gemischt und mit 408 g Ethylacetat verdünnt (Reaktionskomponente 1). ,
10.9 kg 2,04 M (ca. 8900 ml, 18.2 mol) auf 5°C gekühlte Natriumhypochloritlösung (technische Bleichlauge; pH 14) werden bereitgestellt (Reaktionskomponente 2).
Die Reaktionsapparatur besteht aus einem 500-ml-Reaktionsgefäß mit Bodenablass, Mantelkühlung und mechanischem Rührer, das zur Erhöhung der Kühlleistung mit einem externen Kühler verbunden ist, durch den die Reaktionsmischung fortlaufend durchgepumpt und in das Reaktionsgefäss zurückgeführt wird.
In der Apparatur werden 900 ml 0,1 M Phosphatpuffer (pH 7) vorgelegt und auf 5°C gekühlt.
Unter intensiver Rührung werden Reaktionskomponente 1 mit 7,2 g / min (entsprechend 61 mmol 2-Propin-1-ol / min) und Reaktionskomponente 2 mit 91 g / min (entsprechend 152 mmol Hypochlorit / min) in das Reaktionsgefäß gepumpt, aus dem kontinuierlich entsprechende Teile der Reaktionsmischung abgezogen werden.
Dabei wird durch Kühlung die Temperatur zwischen 15 und 20 °C gehalten. Der pH-Wert der Reaktionsmischung wird mit Hilfe eines Titrators, der 25 Gew.%ige Natronlauge fördert, bei 8.5 gehalten.
Eine Analyse der gesammelten Produktlösung zeigt, dass sich insgesamt 469 g (6696 mmol) Propiolsäure gebildet hat (Ausbeute 92 Mol%).

### Beispiel 5 : Oxidation von 2-Butin-1-ol zu 3-Methylpropiolsäure in einem Zweiphasensystem und in Gegenwart von Phasentransferkatalysator

12,3 g (175 mmol) 2-Butin-1-ol werden zusammen mit 1,5 g (8,7 mmol) 4-Hydroxy-TEMPO in 13 g CH₂Cl₂ gelöst (Reaktionskomponente 1).
7,0 g (175 mmol) NaOH werden in 220 g 2,4 M (ca. 180 ml, 432 mmol) Natriumhypochloritlösung (technische Bleichlauge; pH 14) gelöst und auf 5°C gekühlt (Reaktionskomponente 2).
In einem Kolben mit mechanischer Rührung werden 3,5 g (8,7 mmol) Aliquat 336 in 66 ml Wasser und 66 ml CH₂Cl₂ vorgelegt und auf 5°C gekühlt. Reaktionskomponente 1 und 2 werden parallel unter guter Rührung und Kühlung so zugegeben, dass die Innentemperatur nicht über 10 °C steigt. Der pH der Reaktionsmischung wird währenddessen durch Zusatz von 20 Gew%iger Schwefelsäure zwischen pH 8 und pH 10 gehalten.
Nach vollständiger Zugabe wird noch 10 min bei 10°C nachgerührt.
Nach Abtrennung der organischen Phase (enthält 9 Mol% 3-Methylpropanal) wird die wässrige Phase mit Salzsäure (20 Gew.%ig) auf pH 0 eingestellt und 3-mal mit je 50 ml Ethylacetat extrahiert. Die wässrige Phase wird verworfen.
Die Ethylacetat-Phasen werden vereint und ergeben nach teilweiser destillativer Entfernung des Ethylacetats eine ca. 50%ige Lösung von 3-Methylpropiolsäure in Ethylacetat, die 12,1 g (144 mmol)3-Methylpropiolsäure enthält (Ausbeute 82 Mol%).

### Beispiel 6 : Oxidation von 2-Butin-1-ol zu 3-Methylpropiolsäure in einem Zweiphasensystem

12,3 g (175 mmol) 2-Butin-1-ol werden zusammen mit 1,5 g (8,7 mmol) 4-Hydroxy-TEMPO in 13 g CH₂Cl₂ gelöst (Reaktionskomponente 1).
7,0 g (175 mmol) NaOH werden in 220 g 2,4 M (ca. 180 ml, 432 mmol) Natriumhypochloritlösung (technische Bleichlauge; pH 14) gelöst und auf 5°C gekühlt (Reaktionskomponente 2).
In einem Kolben mit mechanischer Rührung werden 66 ml Wasser und 66 ml CH₂Cl₂ vorgelegt und auf 5°C gekühlt. Reaktionskomponente 1 und 2 werden parallel unter guter Rührung und Kühlung so zugegeben, dass die Innentemperatur nicht über 10 °C steigt. Der pH der Reaktionsmischung wird währenddessen durch Zusatz von 20 Gew%iger Schwefelsäure zwischen pH 8 und pH 10 gehalten.
Nach vollständiger Zugabe wird noch 10 min bei 10°C nachgeruhrt.
Nach Abtrennung der organischen Phase (enthält 35 Mol% 3-Methylpropanal) wird die wässrige Phase mit Salzsäure (20 Gew.%ig) auf pH 0 eingestellt und 3-mal mit je 50 ml Ethylacetat extrahiert. Die wässrige Phase wird verworfen.
Die Ethylacetat-Phasen werden vereint und ergeben nach teilweiser destillativer Entfernung des Ethylacetats eine ca. 50%ige Lösung von 3-Methylpropiolsäure in Ethylacetat, die 5,8 g (69 mmol) 3-Methylpropiolsäure enthält (Ausbeute 39 Mol%).

### Beispiel 7 : Oxidation von 2-propin-1-ol zu Propiolsäure in einem wässrigen einphasigen System unter Verwendung einer wässrigen Vorlage und diskontinuierlicher Zugabe der Reaktanden

30 g (535 mmol) 2-Propin-1-ol werden zusammen mit 4,6 g (26,7 mmol) 4-Hydroxy-TEMPO in 30 g Wasser gelöst (Reaktionskomponente 1)
625 g 2,4 M (ca. 500 ml, 1200 mmol) auf 5°C gekühlte Natriumhypochloritlösung (technische Bleichlauge; pH 14) werden bereitgestellt (Reaktionskomponente 2).
In einem 2-l-Glaskolben mit mechanischem Rührer werden 17,8 g (100 mmol) Na₂HPO₄x2H₂O in 800 ml Wasser gelöst, mit Phosphorsäure pH 7 eingestellt und auf 5°C gekühlt. Unter intensiver Rührung und Kühlung werden nun abwechselnd jeweils ca. 45 ml Reaktionskomponente 2 und ca. 6 ml Reaktionskomponente 1 so zugetropft, dass die Innentemperatur nicht über 10 °C steigt. Parallel wird durch fortlaufende Zugabe von 20 Gew%iger Natronlauge der pH-Wert der Reaktionsmischung zwischen 7 und 10 gehalten.
Nach vollständiger Zugabe der Reaktionskomponenten wird noch 10 min nachgerührt. Eine Auswertung des Propiolsäuregehaltes der wassrigen Phase mittels HPLC ergibt eine Rohausbeute von 90 Mol% Propiolsäure.
Die Reaktionsmischung wird mit 300 ml CH₂Cl₂ extrahiert. Nach Abtrennung der organischen Phase wird die wässrige Phase mit Salzsäure (20 Gew.%ig) auf pH 0 eingestellt und 3-mal mit je 300 ml Ethylacetat extrahiert. Die wässrige Phase wird verworfen.
Die Ethylacetat-Phasen werden vereint und ergeben nach teilweiser destillativer Entfernung des Ethylacetats eine ca. 50%ige Lösung von Propiolsäure in Ethylacetat, die 31,2 g (446 mmol) Propiolsäure enthält (Ausbeute 83 Mol%).

### Beispiel 8 : Oxidation von 2-Butin-1,4-diol zu Acetylendicarbonsäure in einem wässrigen einphasigen System mit Acetamido-TEMPO

14,4 g (167 mmol) 2-Butin-1,4-diol werden zusammen mit 2,14 g (10,0 mmol) 4-Acetamido-TEMPO in 94 ml Wasser gelöst (Reaktionskomponente 1).
6,68 g (167 mmol) NaOH werden in 337 ml (0,741 Mol) Natriumhypochloritlösung (ca. 2.2 M technische Bleichlauge; pH 14) gelöst und auf 5°C gekühlt (Reaktionskomponente 2).
In einem Kolben mit mechanischer Rührung werden 50 ml Wasser vorgelegt und auf 3°C gekühlt. Reaktionskomponente 1 und 2 werden parallel unter guter Rührung und Kühlung so zugegeben, dass die Innentemperatur nicht über 10 °C steigt. Der pH-Wert der Reaktionsmischung wird währenddessen durch Zusatz von 20 proz. Natronlauge im Bereich 8,5 bis 10 gehalten. Insgesamt werden ca. 15 ml Natronlauge verbraucht.
Nach vollständiger Zugabe wird noch 20 min nachgerührt.
Die Reaktionsmischung enthält in Lösung 11,4 g Acetylendicarbonsäure und 2,1 g Acetylendicarbonsäure im gebildeten Niederschlag (Gesamtausbeute 13,5 g, 71 %). Es wird mit 300 ml MTBE extrahiert, dann der pH-Wert der wässrigen Reaktionsmischung mit konz. Schwefelsäure unter Rühren im Eisbad auf pH 0 eingestellt und 3-mal mit je 100 ml MTBE ausgeschüttelt. Die MTBE-Extrakte aus der sauren Reaktionsmischung werden eingedampft. Man erhält 11,1 g Acetylendicarbonsäure in Form eines farblosen Feststoffs.

## Patentansprüche

1. Verfahren zur Herstellung von Alkincarbonsäuren, **gekennzeichnet durch** die Oxidation
eines Alkinalkohols
mit einem Hypohalogenit
in Gegenwart einer Nitroxylverbindung
bei einem pH-Wert größer 7
unter fortlaufender Zugabe des Alkinalkohols und des Hypohalogenits zur Reaktionsmischung.

2. Verfahren nach Anspruch 1 , **dadurch gekennzeichnet, dass** die Reaktion in einem Mehrphasensystem durchgeführt wird.

3. Verfahren nach Anspruch 2, **dadurch gekennzeichnet, dass** mindestens ein Phasentransferkatalysator verwendet wird.

4. Verfahren nach Anspruch 1 bis 3, **dadurch gekennzeichnet, dass** die Reaktionsmischung kontinuierlich abgezogen wird.

5. Verfahren nach Anspruche 1 bis 4, **dadurch gekennzeichnet, dass** der pH-Wert der wässrigen Phase der Reaktionsmischung zwischen 7 und 11 liegt.

6. Verfahren nach Anspruche 1 bis 5, **dadurch gekennzeichnet, dass** als Nitroxylverbindung 4-Hydroxy-TEMPO verwendet wird.

7. Verfahren nach Anspruche 1 bis 6, **dadurch gekennzeichnet, dass** die Reaktionstemperatur zwischen - 5°C und 20°C liegt.

8. Verfahren nach Anspruche 1 bis 7, **dadurch gekennzeichnet, dass** 2 bis 3 mol-Aquivalente des Hypohalogenits bezogen auf die Anzahl zu oxidierender funktionaler Gruppen verwendet werden.

9. Verfahren nach Anspruche 1 bis 8, **dadurch gekennzeichnet, dass** als Alkinalkohol 2-Propin-1-ol oder 2-Butin-1,4-diol verwendet wird.

10. Verfahren nach Anspruch 1 bis 9, **dadurch gekennzeichnet, dass** die Umsetzung in Gegenwart von Phosphatpuffer oder Calciumcarbonat durchgeführt wird.

## Claims

1. Process for preparing alkynecarboxylic acids, **characterized by** the oxidation
of an alkyne alcohol
with a hypohalite
in the presence of a nitroxyl compound
at a pH of greater than 7
with continual addition of the alkyne alcohol and of the hypohalite to the reaction mixture.

2. Process according to Claim 1, **characterized in that** the reaction is carried out in a multiphasic system.

3. Process according to Claim 2, **characterized in that** at least one phase transfer catalyst is used.

4. Process according to Claims 1 to 3, **characterized in that** the reaction mixture is removed continuously.

5. Process according to Claims 1 to 4, **characterized in that** the pH of the aqueous phase of the reaction mixture is between 7 and 11.

6. Process according to Claims 1 to 5, **characterized in that** the nitroxyl compound used is 4-hydroxy-TEMPO.

7. Process according to Claims 1 to 6, **characterized in that** the reaction temperature is between -5°C and 20°C.

8. Process according to Claims 1 to 7, **characterized in that** from 2 to 3 mol equivalents of the hypohalite are used based on the number of functional groups to be oxidized.

9. Process according to Claims 1 to 8, **characterized in that** the alkyne alcohol used is 2-propyn-1-ol or 2-butyne-1,4-diol.

10. Process according to Claims 1 to 9, **characterized in that** the reaction is carried out in the presence of phosphate buffer or calcium carbonate.

## Revendications

1. Procédé pour la préparation d'acides carboxyliques acétyléniques, **caractérisé par** l'oxydation d'un alcool acétylénique avec un hypohalogénite en présence d'un composé nitroxyle à un pH supérieur à 7 avec addition continue de l'alcool acétylénique et de l'hypohalogénite au mélange réactionnel.

2. Procédé selon la revendication 1, **caractérisé en ce que** la réaction est réalisée dans un système à plusieurs phases.

3. Procédé selon la revendication 2, **caractérisé en ce qu'**on utilise au moins un catalyseur de transfert de phases.

4. Procédé selon les revendications 1 à 3, **caractérisé en ce que** le mélange réactionnel est soutiré en continu.

5. Procédé selon les revendications 1 à 4, **caractérisé en ce que** le pH de la phase aqueuse du mélange réactionnel est situé entre 7 et 11.

6. Procédé selon les revendications 1 à 5, **caractérisé en ce qu'**on utilise comme composé nitroxyle le 4-hydroxy-TEMPO.

7. Procédé selon les revendications 1 à 6, **caractérisé en ce que** la température de réaction est comprise entre -5°C et 20°C.

8. Procédé selon les revendications 1 à 7, **caractérisé en ce qu'**on utilise 2 à 3 équivalents en mole de l'hypohalogénite par rapport au nombre de groupements fonctionnels à oxyder.

9. Procédé selon les revendications 1 à 8, **caractérisé en ce qu'**on utilise comme alcool acétylénique le 2-propyn-1-ol ou le 2-butyne-1,4-diol.

10. Procédé selon la revendication 1 à 9, **caractérisé en ce qu'**on réalise la transformation en présence de tampon phosphate ou de carbonate de calcium.
